# FASCICULE DE BREVET EUROPEEN

(11) **EP 3 716 913 B1**
(45) Date de publication et mention de la délivrance du brevet: **19.01.2022**
(21) Numéro de dépôt: 18833082.3
(22) Date de dépôt: 27.11.2018
(51) Int. Cl.: A61F 2/30, A61F 2/40

(54) **TIGE HUMÉRALE POUR UN IMPLANT HUMÉRAL DE PROTHÈSE D'ÉPAULE**
OBERARMKNOCHEN-SCHAFT FÜR EINEN HUMERALEN IMPLANTAT VON SCHULTERPROTHESE
HUMERAL STEM FOR A HUMERAL IMPLANT OF SHOULDER PROSTHESIS

(30) Priorité: 28.11.2017 FR 1761302
(43) Date de publication de la demande: 07.10.2020
(73) Titulaire: Shoulder Friends Institute, 75008 Paris (FR)
(72) Inventeur: LEFEBVRE, Yves, 67000 Strasbourg (FR); AUDEBERT, Stéphane, 59268 Blecourt (FR); BARTH, Johannes, 38240 Meylan (FR); CHAROUSSET, Christophe, 75017 Paris (FR); GARRET, Jérôme, 69760 Limonest (FR); GALLINET, David, 25870 Geneuille (FR); GUERY, Jacques, 58000 Nevers (FR); JOUDET, Thierry, 33500 Libourne (FR); GODENECHE, Arnaud, 69450 Saint Cyr Au Mont D'Or (FR)
(74) Mandataire: Germain Maureau
(86) Numéro de dépôt international: PCT/FR2018/052992
(87) Numéro de publication internationale: WO 2019/106276

(56) Documents cités:
- EP-A1- 0 679 375
- WO-A1-2016/094739
- FR-A1- 2 996 442
- US-A1- 2016 278 945

## Description

La présente invention se rapporte à une tige humérale pour un implant huméral de prothèse d'épaule.

Elle se rapporte plus particulièrement à une tige humérale monobloc comprenant :
- une partie diaphysaire de forme allongée, s'étendant selon un axe diaphysaire et conformée pour être implantée dans une cavité médullaire d'un humérus ; et
- une partie métaphysaire conformée pour prendre appui sur une portion métaphysaire réséquée de l'humérus, où la partie métaphysaire se présente sous la forme d'une corolle évasée qui prolonge la partie diaphysaire jusqu'à une face proximale plane centrée sur un axe central, cet axe central étant orthogonal à la face proximale et incliné relativement à l'axe diaphysaire.

Pour un bon ancrage et une bonne tenue de la tige humérale, sa partie métaphysaire prend ainsi appui sur la portion métaphysaire de l'humérus dûment réséquée, et la partie diaphysaire s'ancre dans la cavité médullaire de cet humérus.

Pour répondre à ce besoin d'ancrage, différentes formes plus ou moins satisfaisantes ont été proposées pour la tige humérale, avec par exemple des parties diaphysaires de section transversale ovoïdale ou trapézoïdale ainsi que connu par exemple du document FR 2 996 442.

Cependant, de telles formes ovoïdales ou trapézoïdales pour la partie diaphysaire se poursuivent nécessairement au niveau de la partie métaphysaire qui sera alors plus ou moins ovoïdale ou trapézoïdale, au détriment d'une bonne assise dans la portion métaphysaire réséquée de l'humérus.

L'état de la technique peut également être illustré par l'enseignement du document WO2016/094739 qui décrit une tige humérale monobloc tel que décrite ci-dessus, avec une partie diaphysaire de forme allongée, et une partie métaphysaire se présentant sous la forme d'une corolle évasée qui prolonge la partie diaphysaire jusqu'à une face proximale plane.

La présente invention a pour but de proposer une tige humérale conformée pour offrir une parfaite assise dans la portion métaphysaire réséquée de l'humérus tout en procurant une bonne stabilité de la partie diaphysaire dans la cavité médullaire de l'humérus.

A cet effet, elle propose une tige humérale pour un implant huméral de prothèse d'épaule, ladite tige humérale monobloc comprenant :
- une partie diaphysaire de forme allongée, s'étendant selon un axe diaphysaire et conformée pour être implantée dans une cavité médullaire d'un humérus ;
- une partie métaphysaire conformée pour prendre appui sur une portion métaphysaire réséquée de l'humérus, où la partie métaphysaire se présente sous la forme d'une corolle évasée qui prolonge la partie diaphysaire jusqu'à une face proximale plane centrée sur un axe central, ledit axe central étant orthogonal à ladite face proximale et incliné relativement à l'axe diaphysaire ;
   où la tige humérale selon l'invention est remarquable en ce que :
- la partie diaphysaire présente, orthogonalement à l'axe diaphysaire, une section transversale octogonale à angles chanfreinés convexes ;
- la partie métaphysaire présente, orthogonalement à l'axe central, une section transversale octogonale à angles chanfreinés convexes ;
   de sorte que la tige humérale présente une surface périphérique pourvue de :
   - huit pans latéraux plats qui se prolongent de manière continue de la partie diaphysaire à la partie métaphysaire,
   - huit congés arrondis qui se prolongent de manière continue de la partie diaphysaire à la partie métaphysaire, chaque congé arrondi étant intercalé entre deux pans latéraux adjacents ;
et la tige humérale est également remarquable en ce que, sur la partie métaphysaire, les congés arrondis s'élargissent progressivement et les pans latéraux rétrécissent progressivement de la partie diaphysaire en direction de la face proximale.

Ainsi, la partie diaphysaire de la tige humérale selon l'invention offre, de par sa forme octogonale, une bonne stabilité dans la cavité médullaire de l'humérus. De plus, avec sa partie métaphysaire qui voit progressivement les congés arrondis s'élargirent au détriment des pans latéraux plats, cette partie métaphysaire va présenter une forme substantiellement tronconique, le terme « substantiellement » étant employé car les pans latéraux plats sont présents mais de manière limitée. Grâce à cette forme substantiellement tronconique de la partie métaphysaire, celle-ci offrira une parfaite assise dans la portion métaphysaire réséquée de l'humérus à la fois du côté antérieur, du côté postérieur, du côté médial et du côté latéral. Par ailleurs, un autre intérêt de ces congés arrondis en partie métaphysaire est de diminuer le risque de refend ou de fracture proximale de l'os cortical, notamment lors de l'introduction finale de la tige dans sa cavité médullaire.

Selon une caractéristique, sur la partie métaphysaire, les congés arrondis présentent, tous pour chaque section transversale orthogonale à l'axe central 30, un même rayon de courbure et un même centre de courbure placé sur l'axe central.

Selon une autre caractéristique, les pans latéraux rétrécissent progressivement en direction de la face proximale jusqu'à présenter une largeur proximale inférieure ou égale à 1 millimètre au niveau de la face proximale, afin que les congés arrondis soient sensiblement jointifs pour que ladite face proximale présente un pourtour externe sensiblement cylindrique.

Dans une réalisation particulière, la largeur proximale de chaque pan latéral est nulle de sorte que les congés arrondis sont jointifs pour que la face proximale présente un pourtour externe cylindrique.

Selon une possibilité de l'invention, sur la partie diaphysaire, les congés arrondis sont de largeurs réduites comparativement aux pans latéraux.

Selon une autre possibilité de l'invention, la partie métaphysaire est pourvue d'une cavité ouverte sur la face proximale, ladite cavité présentant un périmètre cylindrique au niveau de la face proximale.

Dans un mode de réalisation particulier, la partie diaphysaire présente une terminaison distale de forme générale arrondie, où les pans latéraux et les congés arrondis se prolongent de manière continue sur cette terminaison distale jusqu'à se rejoindre à un sommet de la terminaison distale.

L'invention se rapporte également à implant huméral de prothèse d'épaule, comprenant une tige humérale conforme à l'invention, et un insert huméral fixé sur la face proximale de la partie métaphysaire et présentant :
- soit une calotte hémisphérique conformée pour une articulation avec une glénosphère d'un implant glénoïdien ;
- soit une tête d'articulation sphérique conformée pour une articulation sur un corps d'articulation d'un implant glénoïdien.

D'autres caractéristiques et avantages de la présente invention apparaîtront à la lecture de la description détaillée ci-après, d'un exemple de mise en œuvre non limitatif, faite en référence aux figures annexées dans lesquelles :

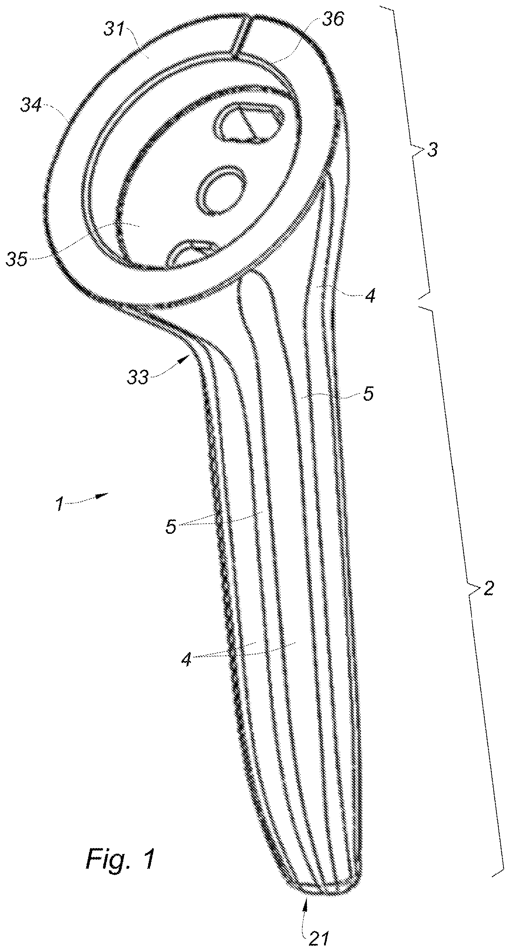

- les figures 1, 2 et 3 sont des vues en perspective d'une tige humérale selon l'invention, selon trois angles de vue distincts ;
- les figures 4 et 5 sont des vues en perspective de la tige humérale des figures 1 à 3 en situation dans un humérus ; et
- les figures 6a, 6b, 6c et 6d sont des vues en coupe de la tige humérale des figures 1 à 3 effectuées selon les plans de coupe respectivement A-A, B-B, C-C et D-D visibles sur la figure 3.

En référence aux figures, une tige humérale 1 comprend une partie diaphysaire 2 de forme allongée, s'étendant selon un axe diaphysaire 20, et présentant une terminaison distale 21 de forme générale arrondie qui se termine par un sommet 22 ; ce sommet 22 pouvant être placé sur l'axe diaphysaire 20 ou pouvant être excentré ou décalé par rapport à l'axe diaphysaire 20 comme c'est le cas dans de mode de réalisation illustré et donné en exemple non limitatif.

La tige humérale 1 comprend en outre une partie métaphysaire 3 qui est monobloc avec la partie diaphysaire 2. Cette partie métaphysaire 3 se présente sous la forme d'une corolle évasée qui prolonge la partie diaphysaire 2 jusqu'à une face proximale 31 plane centrée sur un axe central 30.

Cet axe central 30 est orthogonal à la face proximale 31 et est également incliné relativement à l'axe diaphysaire 20. La partie métaphysaire 3 présente une courbure latérale convexe 32 et une courbure médiale concave 33 dans le prolongement de la partie métaphysaire 3.

La partie métaphysaire 3 est pourvue d'une cavité 35 ouverte sur la face proximale 31 et prévue pour réceptionner un insert huméral (non illustré) qui présente :
- soit une calotte hémisphérique conformée pour une articulation avec une glénosphère d'un implant glénoïdien, dans le cas d'une prothèse d'épaule dite « inversée » ;
- soit une tête d'articulation sphérique conformée pour une articulation sur un corps d'articulation d'un implant glénoïdien, dans le cas d'une prothèse d'épaule dite « anatomique ».

Cette cavité 35 peut être cylindrique ou tronconique et présente, dans les deux cas, un périmètre cylindrique 36 au niveau de la face proximale 31 centré sur l'axe central 30 ; un tel périmètre 36 pouvant éventuellement être chanfreiné.

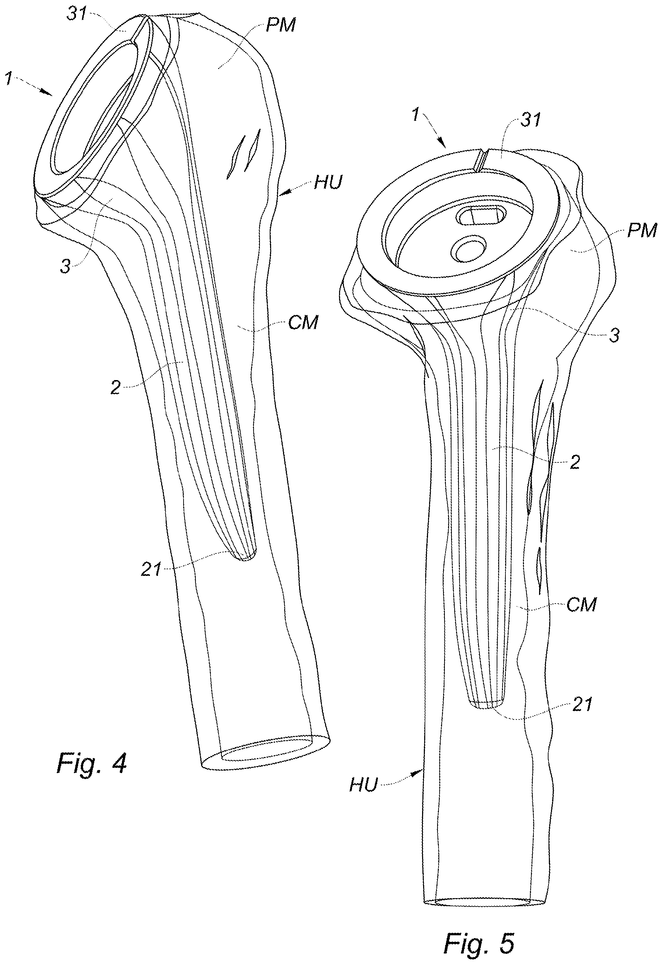

Comme visible sur les figures 4 et 5, la partie diaphysaire 2 est conformée pour être implantée dans une cavité médullaire CM d'un humérus HU, tandis que la partie métaphysaire 3 est conformée pour prendre appui sur une portion métaphysaire PM réséquée de l'humérus HU.

Concernant les formes de la partie diaphysaire 2 et de la partie métaphysaire 3 :
- la partie diaphysaire 2 présente, orthogonalement à l'axe diaphysaire 20 (du sommet 22 jusqu'à la courbure médiale concave 33), une section transversale octogonale à angles chanfreinés convexes en arc de cercle ; et
- la partie métaphysaire 3 présente, orthogonalement à l'axe central 30 (de la courbure médiale concave 33 jusqu'à la face proximale 31), une section transversale octogonale à angles arrondis convexes en arc de cercle.

Ainsi, la tige humérale 1 présente une surface périphérique pourvue de :
- huit pans latéraux 4 plats qui se prolongent de manière continue de la partie diaphysaire 2 à la partie métaphysaire 3, en partant du sommet 22 jusqu'à jusqu'à la face proximale 31 ; et
- huit congés arrondis (ou arqués) 5 qui se prolongent de manière continue de la partie diaphysaire 2 à la partie métaphysaire 3, en partant du sommet 22 jusqu'à jusqu'à la face proximale 31, chaque congé arrondi 5 étant intercalé entre deux pans latéraux 4 adjacents.

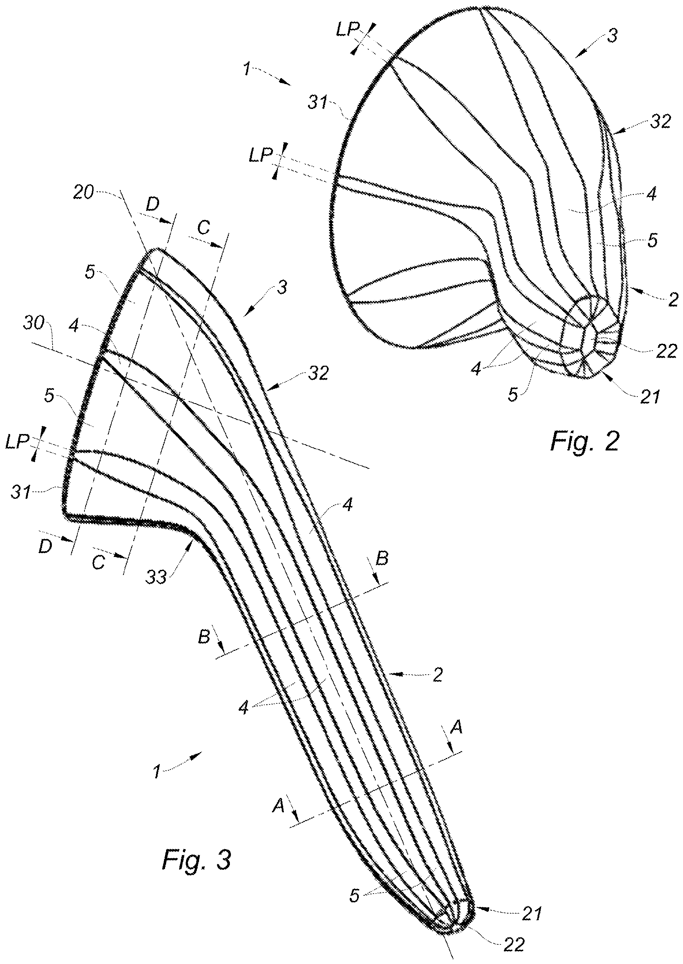

Sur la partie diaphysaire 2, les pans latéraux 4 et les congés arrondis 5 se prolongent de manière continue sur la terminaison distale 21 jusqu'à se rejoindre au sommet 22, ainsi que visible sur les figures 2 et 3.

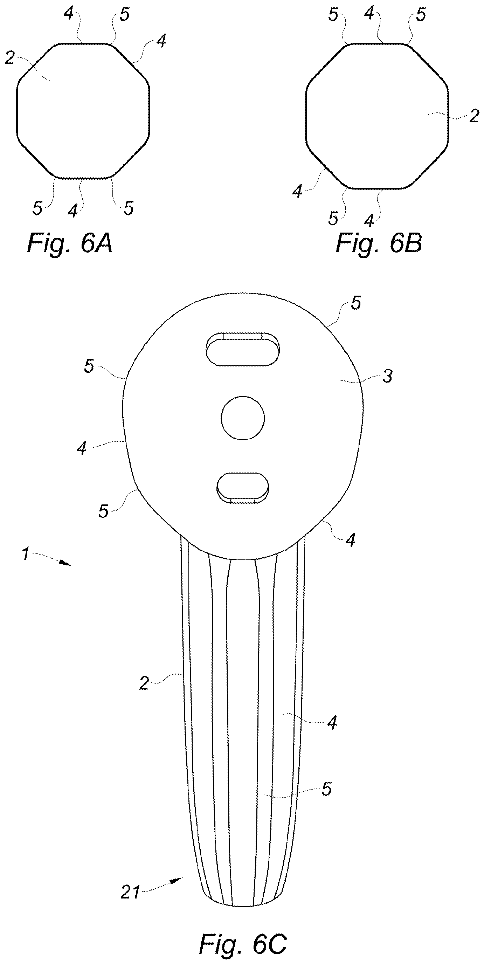

Comme visible notamment sur les figures 6A et 6B, sur la partie diaphysaire 2, les pans latéraux 4 sont plus larges que les congés arrondis 5, et les congés arrondis 5 sont de largeurs sensiblement constantes sauf au niveau de la terminaison distale 21.

Sur la partie diaphysaire 2, les pans latéraux 4 et les congés arrondis 5 rétrécissent au niveau de la terminaison distale 21 en se rapprochant du sommet 22, comme visible sur les figures 2 et 3, du fait de la forme en pointe de la terminaison distale 21.

Sur la partie métaphysaire 3, les congés arrondis 5 présentent tous, pour chaque section transversale orthogonale à l'axe central 30, un même rayon de courbure et un même centre de courbure placé sur l'axe central 30 ; ce rayon de courbure augmentant progressivement de la partie diaphysaire 2 en direction de la face proximale 31.

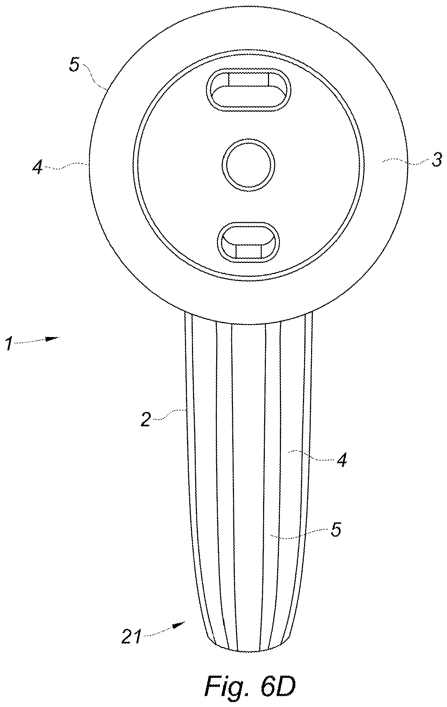

Sur la partie métaphysaire 3, les congés arrondis 5 s'élargissent progressivement de la partie diaphysaire 2 en direction de la face proximale 31 et, à l'inverse, les pans latéraux 4 rétrécissent progressivement de la partie diaphysaire 2 en direction de la face proximale 31, comme visible notamment sur les figures 6C et 6D.

Plus précisément, sur la partie métaphysaire 3, les pans latéraux 4 rétrécissent progressivement en direction de la face proximale 31 jusqu'à présenter une largeur proximale LP inférieure ou égale à 1 millimètre au niveau de la face proximale 31, afin que les congés arrondis 5 soient sensiblement jointifs (aux jeux près que représentent les largeurs proximales LP des huit pans latéraux 4) pour que la face proximale 31 présente un pourtour externe 34 sensiblement cylindrique centré sur l'axe central 30.

Il est à noter que ces largeurs proximales LP peuvent être nulles de sorte que le pourtour externe 34 est strictement cylindrique.

Ainsi, la partie métaphysaire 3 a été présentée ci-dessus comme ayant une section transversale octogonale à angles arrondis (ou arqués) convexes en arc de cercle, ce qui est bien le cas, cependant du fait de l'élargissement des congés arrondis 5 et du rétrécissement des pans latéraux 4, une telle section transversale octogonale à angles chanfreinés convexes pour la partie métaphysaire 3 peut être assimilée à une section transversale cylindrique avec huit pans biseautés (en l'occurrence les pans latéraux 4). Ainsi, la partie métaphysaire 3 est substantiellement tronconique en étant géométriquement dans la continuité de la partie diaphysaire 2.

## Revendications

1. Tige humérale (1) pour un implant huméral de prothèse d'épaule, ladite tige humérale (1) monobloc comprenant :
- une partie diaphysaire (2) de forme allongée, s'étendant selon un axe diaphysaire (20) et conformée pour être implantée dans une cavité médullaire (CM) d'un humérus (HU) ;
- une partie métaphysaire (3) conformée pour prendre appui sur une portion métaphysaire (PM) réséquée de l'humérus (HU), où la partie métaphysaire (3) se présente sous la forme d'une corolle évasée qui prolonge la partie diaphysaire (2) jusqu'à une face proximale (31) plane centrée sur un axe central (30), ledit axe central (30) étant orthogonal à ladite face proximale (31) et incliné relativement à l'axe diaphysaire (20) ; ladite tige humérale (1) étant **caractérisée en ce que** :
- la partie diaphysaire (2) présente, orthogonalement à l'axe diaphysaire (20), une section transversale octogonale à angles arrondis convexes ;
- la partie métaphysaire (3) présente, orthogonalement à l'axe central (30), une section transversale octogonale à angles arrondis convexes ;
de sorte que la tige humérale (1) présente une surface périphérique pourvue de :
- huit pans latéraux (4) plats qui se prolongent de manière continue de la partie diaphysaire (2) à la partie métaphysaire (3),
- huit congés arrondis (5) qui se prolongent de manière continue de la partie diaphysaire (2) à la partie métaphysaire (3), chaque congé arrondi (5) étant intercalé entre deux pans latéraux (4) adjacents ;
**et en ce que,** sur la partie métaphysaire (3), les congés arrondis (5) s'élargissent progressivement et les pans latéraux (4) rétrécissent progressivement de la partie diaphysaire (2) en direction de la face proximale (31).

2. Tige humérale (1) selon la revendication 1, dans laquelle, sur la partie métaphysaire (3), les congés arrondis (5) présentent tous, pour chaque section transversale orthogonale à l'axe central (30), un même rayon de courbure et un même centre de courbure placé sur l'axe central (30).

3. Tige humérale (1) selon l'une quelconque des revendications précédentes, dans laquelle les pans latéraux (4) rétrécissent progressivement en direction de la face proximale (31) jusqu'à présenter une largeur proximale (LP) inférieure ou égale à 1 millimètre au niveau de la face proximale (31), afin que les congés arrondis (5) soient sensiblement jointifs pour que ladite face proximale (31) présente un pourtour externe sensiblement cylindrique.

4. Tige humérale (1) selon la revendication précédente, dans laquelle la largeur proximale (LP) de chaque pan latéral (4) est nulle de sorte que les congés arrondis (5) sont jointifs pour que la face proximale (31) présente un pourtour externe cylindrique.

5. Tige humérale (1) selon l'une quelconque des revendications précédentes, dans laquelle, sur la partie diaphysaire (2), les congés arrondis (5) sont de largeurs réduites comparativement aux pans latéraux (4).

6. Tige humérale (1) selon l'une quelconque des revendications précédentes, dans laquelle la partie métaphysaire (3) est pourvue d'une cavité (35) ouverte sur la face proximale (31), ladite cavité (35) présentant un périmètre cylindrique (36) au niveau de la face proximale (31).

7. Tige humérale (1) selon les revendications l'une quelconque des revendications précédentes, dans laquelle la partie diaphysaire (2) présente une terminaison distale (21) de forme générale arrondie, où les pans latéraux (4) et les congés arrondis (5) se prolongent de manière continue sur cette terminaison distale (21) jusqu'à se rejoindre à un sommet (22) de la terminaison distale (21).

8. Implant huméral de prothèse d'épaule, comprenant une tige humérale (1) conforme à l'une quelconque des revendications précédentes, et un insert huméral fixé sur la face proximale (31) de la partie métaphysaire (3) et présentant :
- soit une calotte hémisphérique conformée pour une articulation avec une glénosphère d'un implant glénoïdien ;
- soit une tête d'articulation sphérique conformée pour une articulation sur un corps d'articulation d'un implant glénoïdien.

## Patentansprüche

1. Humeraler Schaft (1) für ein humerales Schulterprothesenimplantat, wobei der einstückige humerale Schaft (1) umfasst:
- einen länglichen diaphysären Teil (2), der sich gemäß einer diaphysären Achse (20) erstreckt und ausgebildet ist, um in einen medullären Hohlraum (CM) eines Humerus (HU) implantiert zu sein;
- einen metaphysären Teil (3), der ausgebildet ist, um sich auf einem resezierten metaphysären Abschnitt (PM) des Humerus (HU) abzustützen, wobei der metaphysäre Teil (3) in Form einer erweiterten Krone vorliegt, die den diaphysären Teil (2) bis zu einer ebenen proximalen Fläche (31) verlängert, die auf einer zentralen Achse (30) zentriert ist, wobei die zentrale Achse (30) zur proximalen Fläche (31) orthogonal und zur diaphysären Achse (20) relativ geneigt ist;
wobei der humerale Schaft (1) **dadurch gekennzeichnet ist, dass**:
- der diaphysäre Teil (2) orthogonal zur diaphysären Achse (20) einen achteckigen Querschnitt mit konvexen abgerundeten Ecken aufweist;
- der metaphysäre Teil (3) orthogonal zur zentralen Achse (30) einen achteckigen Querschnitt mit konvexen abgerundeten Ecken aufweist;
so dass der humerale Schaft (1) eine periphere Oberfläche aufweist, die versehen ist mit:
- acht flachen Seitenwangen (4), die sich kontinuierlich vom diaphysären Teil (2) zum metaphysären Teil (3) verlängern,
- acht abgerundeten Hohlkehlen (5), die sich kontinuierlich vom diaphysären Teil (2) zum metaphysären Teil (3) verlängern, wobei sich jede abgerundete Hohlkehle (5) zwischen zwei benachbarten Seitenwangen (4) befindet;
und dass sich auf dem metaphysären Teil (3) die abgerundeten Hohlkehlen (5) nach und nach verbreitern und sich die Seitenwangen (4) vom diaphysären Teil (2) in Richtung der proximalen Fläche (31) nach und nach verschmälern.

2. Humeraler Schaft (1) nach Anspruch 1, wobei auf dem metaphysären Teil (3) die abgerundeten Hohlkehlen (5) alle für jeden zur zentralen Achse (30) orthogonalen Durchmesser denselben Krümmungsradius und dasselbe Krümmungszentrum auf der zentralen Achse (30) aufweisen.

3. Humeraler Schaft (1) nach einem der vorangehenden Ansprüche, wobei sich die Seitenwangen (4) nach und nach in Richtung der proximalen Fläche (31) verschmälern, bis sie eine proximale Breite (LP) von unter oder gleich 1 Millimeter im Bereich der proximalen Fläche (31) aufweisen, damit die abgerundeten Hohlkehlen (5) etwa anliegend sind, damit die proximale Fläche (31) einen etwa zylindrischen äußeren Umfang aufweist.

4. Humeraler Schaft (1) nach vorangehendem Anspruch, wobei die proximale Breite (LP) jeder Seitenwange (4) null ist, so dass die abgerundeten Hohlkehlen (5) anliegend sind, damit die proximale Fläche (31) einen zylindrischen äußeren Umfang aufweist.

5. Humeraler Schaft (1) nach einem der vorangehenden Ansprüche, wobei auf dem diaphysären Teil (2) die abgerundeten Hohlkehlen (5) im Vergleich zu den Seitenwangen (4) reduzierte Breiten haben.

6. Humeraler Schaft (1) nach einem der vorangehenden Ansprüche, wobei der metaphysäre Teil (3) mit einem auf der proximalen Fläche (31) offenen Hohlraum (35) versehen ist, wobei der Hohlraum (35) im Bereich der proximalen Fläche (31) zylindrischen Umkreis (36) aufweist.

7. Humeraler Schaft (1) nach einem der vorangehenden Ansprüche, wobei der diaphysäre Teil (2) einen allgemein abrundeten distalen Abschluss (21) aufweist, wobei sich die Seitenwangen (4) und die abgerundeten Hohlkehlen (5) auf diesem distalen Abschluss (21) kontinuierlich verlängern, bis sie sich an einer Spitze (22) des distalen Abschlusses (21) treffen.

8. Humerales Schulterprothesenimplantat, umfassend einen humeralen Schaft (1) nach einem der vorangehenden Ansprüche, und einen auf der proximalen Fläche (31) des metaphysären Teils (3) befestigten humeralen Einsatz und aufweisend:
- entweder eine halbkugelige Kalotte, die für ein Gelenk ausgebildet ist, mit einer Kugelpfanne eines Gelenkpfannenimplantat;
- oder einen kugeligen Gelenkkopf, der für ein Gelenk ausgebildet ist, auf einem Gelenkkörper eines Gelenkpfannenimplantats.

## Claims

1. A humeral rod (1) for a humeral implant of a shoulder prosthesis, said integral humeral rod (1) comprising:
- an elongate-shaped diaphyseal portion (2), extending along a diaphyseal axis (20) and shaped so as to be implanted in a medullary cavity (CM) of a humerus (HU);
- a metaphyseal portion (3) shaped so as to bear on a resected metaphyseal portion (PM) of the humerus (HU), wherein the metaphyseal portion (3) is in the form of a flared corolla which extends the diaphyseal portion (2) up to a planar proximal face (31) centered on a central axis (30), said central axis (30) being orthogonal to said proximal face (31) and inclined with respect to the diaphyseal axis (20);
said humeral rod (1) being **characterized in that:**
- the diaphyseal portion (2) has, orthogonally to the diaphyseal axis (20), an octagonal cross-section with convex rounded angles;
- the metaphyseal portion (3) has, orthogonally to the central axis (30), an octagonal cross-section with convex rounded angles;
so that the humeral rod (1) has a peripheral surface provided with:
- eight flat lateral facets (4) which continuously extend from the diaphyseal portion (2) to the metaphyseal portion (3),
- eight rounded fillets (5) which continuously extend from the diaphyseal portion (2) to the metaphyseal portion (3), each rounded fillet (5) being interposed between two adjacent lateral facets (4);
**and in that,** on the metaphyseal portion (3), the rounded fillets (5) progressively widen and the lateral facets (4) progressively narrow from the diaphyseal portion (2) in the direction of the proximal face (31).

2. The humeral rod (1) according to claim 1, wherein, on the metaphyseal portion (3), all of the rounded fillets (5) have, for each cross-section orthogonal to the central axis (30), a same radius of curvature and a same center of curvature placed on the central axis (30).

3. The humeral rod (1) according to any one of the preceding claims, wherein the lateral facets (4) progressively narrow in the direction of the proximal face (31) until having a proximal width (LP) smaller than or equal to 1 millimeter at the level of the proximal face (31), so that the rounded fillets (5) could be substantially matched so that said proximal face (31) has a substantially cylindrical outer circumference.

4. The humeral rod (1) according to the preceding claim, wherein the proximal width (LP) of each lateral facet (4) is zero so that the rounded fillets (5) could be matched so that the proximal face (31) has a cylindrical outer circumference.

5. The humeral rod (1) according to any one of the preceding claims, wherein, on the diaphyseal portion (2), the rounded fillets (5) have reduced widths in comparison with the lateral facets (4).

6. The humeral rod (1) according to any one of the preceding claims, wherein the metaphyseal portion (3) is provided with a cavity (35) open onto the proximal face (31), said cavity (35) having a cylindrical perimeter (36) at the level of the proximal face (31).

7. The humeral rod (1) according to any one of the preceding claims, wherein the diaphyseal portion (2) has a distal end (21) with a rounded general shape, where the lateral facets (4) and the rounded fillets (5) continuously extend over this distal end (21) until meeting at an apex (22) of the distal end (21).

8. A humeral implant of a shoulder prosthesis, comprising a humeral rod (1) in accordance with any one of the preceding claims, and a humeral insert fastened on the proximal face (31) of the metaphyseal portion (3) and having:
- either a hemispherical cap shaped for articulation with a glenosphere of a glenoid implant;
- or a spherical articulation head shaped for articulation on an articulation body of a glenoid implant.
